(19) Europäisches Patentamt

European Patent Office

Office européen des brevets

(11) **EP 1 008 358 B1**

## (12) EUROPEAN PATENT SPECIFICATION

(45) Date of publication and mention
of the grant of the patent:
**18.09.2002 Bulletin 2002/38**

(51) Int Cl.[7]: **A61M 5/165**

(21) Application number: **99123376.8**

(22) Date of filing: **23.11.1999**

(54) **Infusion filter**

Infusionsfilter

Filtre de perfusion

(84) Designated Contracting States:
**DE FR GB IT SE**

(30) Priority: **09.12.1998 JP 34983698**

(43) Date of publication of application:
**14.06.2000 Bulletin 2000/24**

(73) Proprietor: **JMS Co., Ltd.**
**Hiroshima-shi, Hiroshima 730-8652 (JP)**

(72) Inventor: **Nakashima, Masakuni,**
**c/o JMS CO., LTD.**
**Hiroshima-shi, Hiroshima 730-8652 (JP)**

(74) Representative:
**Schwarzensteiner, Marie-Luise, Dr. et al**
**Grape & Schwarzensteiner**
**Patentanwälte**
**Sebastiansplatz 7**
**80331 München (DE)**

(56) References cited:
**EP-A- 0 492 634          WO-A-87/06845**
**WO-A-90/11812          US-A- 4 267 053**
**US-A- 4 568 366          US-A- 4 695 382**

**Description**

The present invention relates to a filter for removing foreign substances that are contained in a liquid medicine and not suitable for the living body when the liquid medicine is infused into a patient.

In medical facilities, infusion is widely performed for the purpose of nutrition supplement, regulation of electrolyte balance inside the body, water supplement, medical treatments, and the like. When infusion is performed, foreign substances may get mixed in a liquid medicine of infusion. Examples of such foreign substances include: foreign substances contained in a liquid medicine from the beginning, cutting dust mixed when a rubber cap of an infusion container is pierced with a needle, fine particles of glass dust, bacteria entering a liquid medicine when an infusion set is prepared or when a plurality of liquid medicines are blended, and the like. Since such foreign substances are harmful to the human body, they should be inhibited from being infused into patients. Therefore, an infusion filter often is used along with an infusion set for the purpose of removing the above-mentioned foreign substances.

US-A-4,695,382 discloses an inline final filter unit, wherein a flexible, non-porous intravenous tubing member contains microporous hollow fibers arranged within the tubular member parallel to the longitudinal direction of the tubing. The fibers are closed at one end and open at the opposite end. A flow blocking material is arranged around the fibers.

WO-A-87/06845 discloses an infusion filter device, wherein longitudinally positive hollow filter fibers and a flow blocking material are arranged within a tubular body for directing flow through the material of the hollow fibers.

US-A-4,267,053 discloses a filter including at least one porous hollow fiber arranged within the casing parallel to the longitudinal direction thereof. The ratio of the total effective filtration area of the hollow filter to the capacity of the casing is about 4:1.

US-A-4,568,366 discloses a bundle of filter tubes having outer diameters of no more than (0,02 inch) 0,5 mm. The filter tubes are folded.

Examples of the infusion filter include a flat membrane infusion filter, a hollow fiber infusion filter, etc. Each filter has its own feature. Recently, much attention is directed to the hollow fiber infusion filter because of the below mentioned reasons ① and ②, among others.

① A hollow fiber infusion filter can remove fine particles almost perfectly.
② A hollow fiber infusion filter can secure a large membrane area even when an amount of the packed membrane is small.

Desirable requirements for an infusion filter include the below mentioned three requirements.

(1) The flow rate of filtration is large.
(2) The amount of membrane packed in a filter ("a priming amount" is also referred to) is small.
(3) The area of the filter membrane is small.

When the flow rate of filtration is large, the permeability of the infusion filter is good and the filtering capacity per unit time is excellent. When the amount of packed membrane is small, a dead volume (a retention volume) of the liquid medicine residing in a housing of the infusion filter is small. This is particularly advantageous when a trace amount of the liquid medicine is administered or specific liquid medicine is rapidly infused into the body. In addition, the amount of liquid residing in the infusion filter after the infusion is completed can be reduced. When the area of the filter membrane is small, the material cost can be reduced. Thus, it is advantageous with regard to manufacturing cost. Furthermore, by reducing the area of the membrane, the amount of the packed membrane can be reduced, thus reducing the amount of the liquid medicine adsorbed to the filter.

However, the above-mentioned requirement (1) is contradictory to the requirements (2) and (3). Therefore, it has not been easy to manufacture an infusion filter having an excellent property. Furthermore, it has been further difficult to manufacture such infusion filters at low cost. For example, when a hollow fiber membrane infusion filter is used, in order to increase the flow rate of filtration by the whole infusion filter, it is necessary to increase the membrane area of the hollow fiber of the filter. In order to increase the membrane area of the hollow fiber, larger amount of hollow fiber membranes is required. Furthermore, in order to pack the larger amount of hollow fiber membranes, a large-size filter housing is required. As a result, the amount of the packed membrane in the infusion filter is increased. On the contrary, in order to reduce the membrane area or the amount of packed membrane in the infusion filter, the requirements with regard to the flow rate of filtration cannot be realized.

As mentioned above, the above-mentioned three requirements are contradicting to each other, thus making it difficult to manufacture an infusion filter having an excellent property at low cost.

It is therefore an object of the present invention to provide an infusion filter having an excellent property at low cost.

In order to achieve the above-mentioned object, the infusion filter of the present invention comprises the features of claims 1.

It is preferable in the above-mentioned infusion filter that the packing rate is in the range from 15 to 35 %.

Furthermore, it is preferred that the effective length of a filtration portion substantially capable of filtration of the porous hollow fiber is in the range from 2.5 to 3.5 cm, and specifically preferably in the range from 2.5 to 3.0 cm.

Furthermore, it is preferable in the above-mentioned infusion filter that an average inner diameter of the hollow fibers forming the hollow fiber bundle is in the range from 100 to 500 $\mu$m and an average thickness of the hollow fibers is in the range from 20 to 200 $\mu$m. More preferably, the average inner diameter is in the range from 200 to 400 $\mu$m and the average thickness is in the range from 50 to 150 $\mu$m.

Furthermore, it is preferable in the above-mentioned infusion filter that the hollow fiber comprises any of materials selected from the group consisting of polysulfone, polyethersulfone, polypropylene, polyethylene, cellulose, cellulose derivative, polyacrylonitrile, ethylene-vinyl acetate copolymer and ethylene vinyl alcohol. More preferably, the hollow fiber is a hydrophilic material so that it easily matches with an infusion liquid.

Furthermore, it is preferable in the above-mentioned infusion filter that the number of the hollow fibers packed in the housing is in the range from 10 to 50. More preferably, the number is in the range from 10 to 30.

Furthermore, it is preferable in the above-mentioned infusion filter that the amount of liquid filled in the housing is 3.0ml or less. More preferably, the amount is in the range from 1.0 to 2.0 ml.

Furthermore, it is preferable in the above-mentioned infusion filter that the housing is cylindrical shape having a length of 2.0 to 5.0 cm and an inner diameter of 0.3 to 2.0 cm. More preferably, the length of the cylinder is in the range from 2.0 to 3.0 cm and the inner diameter is in the range from 0.5 to 1.5 cm.

Furthermore, it is preferable in the above-mentioned infusion filter that the flow rate of filtration through the filtration portion is in the range from 15 to 50 ml/min.

Furthermore, it is preferable in the above-mentioned infusion filter that the total effective filtration area of the filtration portion is in the range from 10 to 40 cm$^2$. More preferably, the area is in the range from 10 to 25 cm$^2$.

Furthermore, it is preferable in the above-mentioned infusion filter that the hollow fibers packed in the housing have substantially the same length.

FIG. 1 is a graph showing the influence of the effective length of the hollow fiber on the flux or cost in an infusion filter of Example 1 of the present invention.

FIG. 2 is a graph showing the influence of the packing rate on the flux or a filling amount in an infusion filter of Example 1 of the present invention.

FIG. 3 is a schematic view showing an infusion filter in one embodiment of the present invention.

FIG. 4 is a graph showing the resultant packing rate and flux when the effective length of the hollow fiber is 2.5cm in Example 3 of the present invention.

The present invention solved the problems of the prior art by properly balancing the above-mentioned three requirements: namely, (1) the improvement of the flow rate of filtration, (2) the reduction of the amount of packed membrane, and (3) the reduction of a membrane area. More specifically, by setting the rate of packing the hollow fibers in an infusion filter (hereinafter, "a packing rate" will also be referred to) at a predetermined range, and by keeping the amount of the packed membrane at a specific amount, the flow rate of filtration was improved. Furthermore, by setting the effective length of the hollow fiber at a predetermined range, the flow rate of filtration was improved and the membrane area was reduced. The effective length herein is defined as a length in an axial direction of the region (portion) substantially capable of filtering liquid of the hollow fiber. Furthermore, by setting the effective length of the hollow fiber and the packing rate at predetermined ranges respectively, an infusion filter having a more excellent property was provided at low cost.

In particular, when the effective length is set at 4.5 cm or less, the packing rate of the hollow fibers is preferably 15 to 40 %, more preferably 15 to 35 %, and furthermore preferably 20 to 30 % so as to satisfy the conditions: the flow rate of filtration is 23ml/min or more and the amount of filled liquid is 1.5 ml or less.

Furthermore, in the present invention, the various kinds of embodiments described below can be employed.

(1) An average inner diameter of the hollow fibers forming the hollow fiber bundle is 100 to 500 $\mu$m and an average wall thickness of the hollow fibers is 20 to 200 $\mu$m.

(2) The above-mentioned hollow fiber is selected from any of synthetic resins of polysulfone (PS), polyethersulfone (PES), polypropylene (PP), polyethylene (PE), cellulose, cellulose derivative, polyacrylonitrile (PAN), ethylene-vinyl acetate copolymer (EVA) and ethylene vinyl alcohol (EVAL).

(3) The effective length is 2.0 to 4.5 cm, preferably 2.5 to 3.5 cm, and more preferably 2.5 to 3.0 cm.

(4) The number of the hollow fibers packed in the housing is 10 to 50.

(5) The amount of liquid filled in the housing is 3.0 ml or less.

(6) The housing has a cylindrical shape having a length of 2.0 to 5.0 cm and an inner diameter of 0.3 to 2.0 cm.

(7) The flow rate of filtration through the filtration portion is 15 to 50 ml/min.

(8) The total effective filtration area of the filtration portion is 10 to 40 cm$^2$.

Example

Hereinafter, an influence of the constituent features of the present invention on the property or the manufacturing cost of an infusion filter will be described by way of examples.

The packing rate of the hollow fibers in the following examples was calculated from the following equation 1. More specifically, the equation 1 means that the ratio of the entire cross-sectional area of the hollow fibers with respect to the cross-sectional area of the housing at, for example, a cross section of the boundary between the hollow fiber and the potting material.

$$(\text{equation 1}) \qquad \text{packing rate}(\%) = \frac{\pi r^2 \times \text{number of hollow fibers} \times 2}{\pi R^2} \times 100$$

wherein r denotes an outside radius of the hollow fiber; R denotes an inside radius of the housing; and the number of the hollow fibers is the number before they are folded in half.

Example 1

a. Materials of hollow fibers

(1) Hollow fiber: Porous hollow fibers (produced by Membra (old firm name: AKZO) ) made of polyethersulfone, having an inner diameter of 300μ m, a wall thickness of 100μm, an average pore diameter of 0.2 μm, maximum pore diameter of 0.6μm, and the length in an axial direction of 6.0cm, 7.0cm, 8.0cm, 9.0cm, 10.0cm and 11.0cm respectively were used. Each porous hollow fiber was fixed with polyurethane resin at both ends by potting. Thus, the hollow fibers having the effective length of 2.0cm, 2.5cm, 3.0cm, 3.5cm 4.0cm and 4.5cm were respectively prepared. The effective length herein denotes a length in an axial direction of the portion substantially capable of filtration.

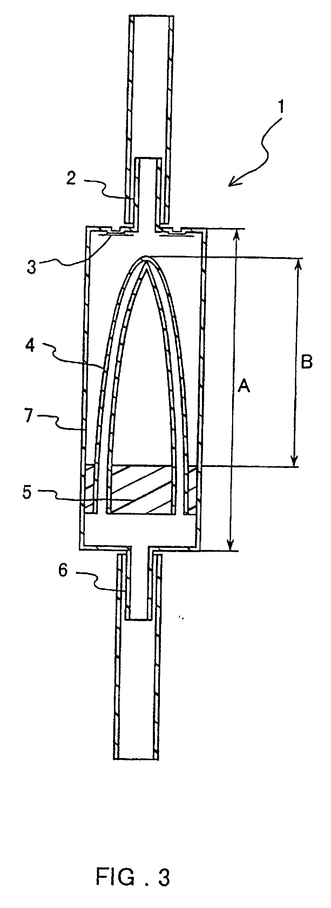

(2) Housing: A transparent cylindrical shaped housing made of polymethyl methacrylate having an inner diameter of 0.5cm and a length of each of the above-mentioned effective length + 0.5cm. FIG. 3 shows its schematic view. In FIG. 3, reference numeral 1 denotes an infusion filter in one embodiment of the present invention; 2 denotes a liquid inlet port for liquid to be filtered; 3 denotes an air vent; 4 denotes a hollow fiber for filtering liquid; 5 denotes a tube seal (potting material) for sealing the outside of the both ends of the hollow fiber 4; 6 denotes a liquid outlet port for taking out the filtered liquid; and 7 denotes a housing. The reference mark A shows a length of the housing 7 and B is a region of the hollow fiber capable of filtration (a region that is a reference for the effective length).

Herein, since the hollow fiber is folded in half at an inflection point at the uppermost part, the liquid flow is interrupted. Moreover, even if the inflection point is in liquid communication, the length of the passage corresponds to that of one way as shown in B of FIG. 3, because liquid is supplied from the both ends of the hollow fibers.

The liquid to be filtered is supplied from the liquid inlet port 2 into the inside of the housing 7, enters the inside of the hollow fiber 4 while being filtered and cleaned when the liquid passes through the hollow fiber 4, passes through the inside of the hollow fiber 4 present at the potting material 5, and is taken out from the end of the hollow fiber at the liquid outlet port. The not-filtered product that failed to pass through the hollow fiber 4 remains inside the housing 7.

(3) Infusion set equipped with an infusion filter: infusion set 216D produced by JMS CO., LTD.

(4) Drug solution: "Aminotripa II" (trade name of a product produced by Otsuka Pharmaceutical Co., Ltd.).

b. Method

(1) The above-mentioned liquid medicine was allowed to flow with head of 90cm (this numerical value is determined based on the general application mode in hospitals) by using an infusion set equipped with a different effective length of the infusion filter.

(2) The membrane area having the hollow fiber of each effective length was calculated. Then, the flux was measured from the flow rate of filtration of the filtered liquid when the liquid was allowed to flow by the above-mentioned method. The measurement conditions and the results are shown in Table 1.

Table 1

| Effective length (cm) | 2.0 | 2.5 | 3.0 | 3.5 | 4.0 | 4.5 |
|---|---|---|---|---|---|---|
| Packing rate of hollow fibers (vol.%) | 40 | 40 | 40 | 40 | 40 | 40 |
| Membrane area (cm$^2$) | 12.6 | 15.7 | 18.8 | 22.0 | 25.1 | 28.3 |
| Flow rate of filtration (ml/min) | 19.6 | 23.4 | 25.4 | 28.0 | 29.7 | 30.4 |
| Flux (ml/min • cm$^2$) | 1.43 | 1.37 | 1.27 | 1.17 | 1.08 | 0.99 |
| [n=10, the flow rate of filtration and the flux are shown as average values] | | | | | | |

(3) Table 2 shows a structure of the filter that satisfies the targeted flow rate of filtration (23 ml/min) calculated from the flux of Table 1.

Table 2

| Effective length (cm) | 2.0 | 2.5 | 3.0 | 3.5 | 4.0 | 4.5 |
|---|---|---|---|---|---|---|
| Necessary area of membrane (cm$^2$) | 16.1 | 16.8 | 18.6 | 19.7 | 21.2 | 23.3 |
| Number of hollow fibers (number) | 51 | 43 | 39 | 36 | 34 | 33 |
| Cost (yen/hollow fiber) | 17.14 | 16.51 | 16.85 | 17.28 | 17.95 | 19.01 |

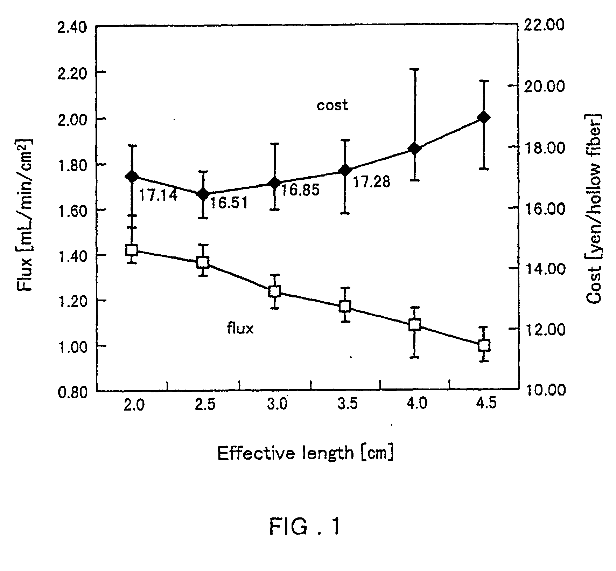

(4) Based on Table 2, the effective length of the hollow fiber is plotted against the flux and plotted against the cost respectively in a graph in FIG. 1. In this graph, an abscissa shows the effective length of the hollow fiber, and an ordinate shows the flux that is a reference for the filtered amount and the cost per hollow fiber. Thus, the relationship between the effective length of the hollow fiber of the infusion filter that satisfies the targeted flow rate of filtration (23ml/min), the flux and the cost was investigated. The unit of the flux is ml/min · cm$^2$. The flow rate of filtration is expressed as the product of the flux and the effective membrane area of the hollow fiber. The unit of the cost is yen/hollow fiber. Within the range measured from FIG. 1, as the length of the hollow fiber, i.e. the effective length of the hollow fiber, becomes shorter, the flux becomes larger, thus allowing liquid to flow easily. Therefore, in this graph in which the flow rate of filtration is constant, as the flux is larger, the membrane area becomes smaller. Therefore, as the effective length of the hollow fiber becomes shorter, the area of the hollow fiber membrane for obtaining the specified flow rate of filtration can be reduced.

As a result, the cost of the hollow fiber can be reduced. Actually, a graph of FIG. 1 shows that in the range where the effective length of the hollow fiber is reduced from 4.5 to 2.5cm, the shorter the length is, the cost per hollow fiber is reduced. However, in the range where the effective length of the hollow fiber is reduced from 2.5 to 2.0cm, the shorter the effective length is, the cost is contrarily increased. The reason of this is probably because the non-filtering surface is increased with respect to the effective filtration surface of the hollow fiber due to the reduction of the effective length. In the hollow fiber infusion filter, the both ends of the hollow fiber are fixed with a potting material, and the portion of the hollow fiber that is fixed with the potting material is a non-filtering surface that cannot filter liquid. When the length of the hollow fiber is reduced, the ratio of the non-filtering surface with respect to the entire hollow fiber is increased. Consequently, the cost is contrarily increased. Therefore, from the above-mentioned results, in order to obtain an infusion filter having an excellent property, hollow fibers having the effective length of 2.0 to 4.5 cm practically can be used. From the viewpoint of the cost, the effective length of the hollow fibers is preferably 2.5 to 3.5cm, more preferably 2.5 to 3.0cm.

Example 2

a. Materials of hollow fibers

The hollow fiber is the same as Example 1 except that the effective length is set at 4.5cm and the packing rates (vol.%) of the hollow fibers packed in the housing with an inner diameter of 5.0 mm are changed to 10%, 20%, 30%, 40% and 50%.

b. Method

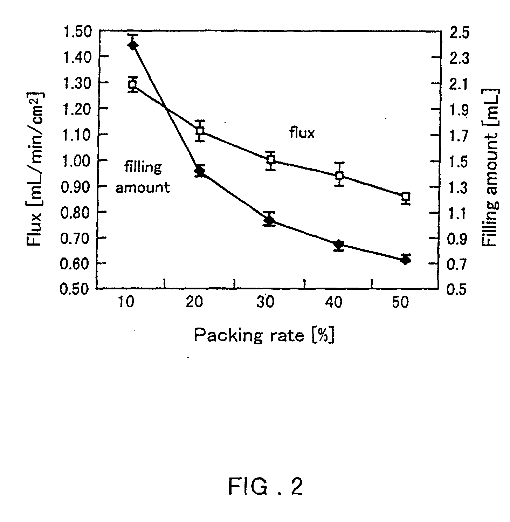

(1) An infusion set equipped with an infusion filter having a different packing rate was used and the liquid medicine of Example 1 was allowed to flow.
(2) The flow rate of filtration through the infusion filter having respective packing rates was measured and the flux was calculated.
(3) A structure of the filter for satisfying the targeted flow rate of filtration (23 ml/min) calculated from the flux was determined. Then, the packing rate of the hollow fiber was plotted against the flux and plotted against the filling amount respectively in a graph (FIG. 2).

FIG. 2 is a graph showing the data obtained as a result of performing infusion with keeping the effective length constant and changing the packing rates. In the graph, the abscissa shows the packing rate (%) of the hollow fibers packed in the housing, and the ordinate shows the flux that is a reference for the flow rate of filtration and the amount of packed infusion filter. Thus, the relationship between the packing rate that satisfies the targeted flow rate of filtration (23 ml/min), the flux and the filling amount was investigated. The unit of the flux is the same as in Example 1, and the unit of the filling amount is ml. In the range measured from FIG. 2, as the filling amount is reduced, the flux is improved. However, the filling amount for obtaining the targeted flow rate of filtration is accordingly increased. The reason why the flux is improved when the packing rate is reduced is thought to be because the space between the hollow fibers are increased as the packing rate is smaller and thereby the disturbance of flow due to overlapping of the hollow fibers is reduced, thus increasing the flux. Furthermore, in order to achieve the targeted flow rate of filtration, in a case where the same amount of the hollow fibers are used, the filling amount (volume of the housing) is increased by reducing the packing rate. Thus, in a case where the length of the hollow fiber is made constant, the reduction of the packing rate improves the flux and increases the filling amount. In other words, the reduction of the packing rate provides two contradicting effects on the property of the infusion filter. Therefore, the packing rate is determined by determining the permissible maximum value or minimum value of the both items (flux and filling amount). For example, in a graph of FIG. 2, when the permissible filling amount is 1.5ml or less, the packing rate providing the filling amount of less than 1.5ml is 20 to 50 %. In order to obtain a maximum flux within the range, the packing rate is 20%. As mentioned above, a suitable range of the packing rate of the hollow fiber is selected in accordance with the value of the permissible flux or filling amount. However, the range is preferably 15 to 40%, more preferably 15 to 35% and furthermore preferably 20 to 30%.

Example 3

The same experiment as Example 2 was carried out except that the effective length of the hollow fiber was changed to 2.5cm.
Table 3 shows the resultant packing rate and flux when the effective length of the hollow fiber was 2.5cm.

Table 3

| Packing rate of hollow fiber (vol.%) | 10 | 20 | 30 | 40 | 50 |
|---|---|---|---|---|---|
| Flux (ml/min • cm$^2$) | 1.42 | 1.26 | 1.16 | 1.03 | 0.98 |
| [n=10, the flux is shown as an average value] | | | | | |

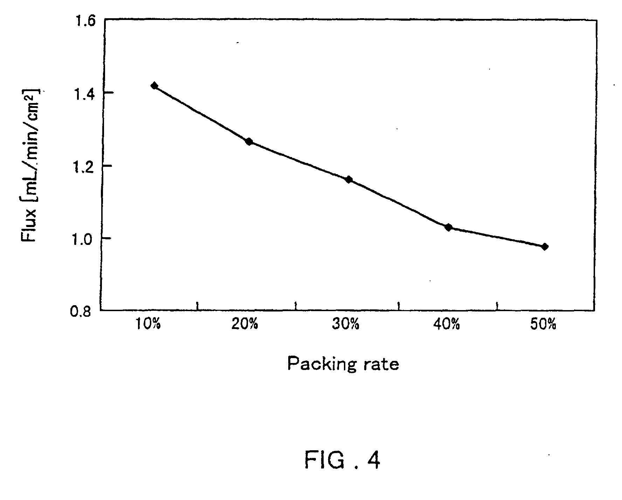

Furthermore, FIG. 4 is a graph showing the results of Table 3. As is apparent from Table 3 and FIG. 4, the packing rate of the hollow fiber was preferably 15 to 40%.
As mentioned above, the hollow fiber infusion filter of the example of the present invention can provide the following effects.

(1) A large flow rate of filtration amount can be realized. Therefore, the amount of liquid to be filtered per minute is increased. Furthermore, the infusion can be performed under a small pressure head.
(2) The retention amount is small because the packing amount is small. As a result, the problem can be solved in terms of the retention of a trace amount of liquid medicine or the lag time of drug efficacy of fast-acting medicine, and the like.
(3) The area of the filter membrane can be reduced. As a result, the manufacturing cost of the infusion filter can be reduced, providing the filter at low cost.

**Claims**

1. An infusion filter comprising a housing having a liquid inlet port and a liquid outlet port, said housing being packed with a bundle of porous hollow fibers with the outside of the both ends fixed with potting materials, said porous hollow fibers provided between said liquid inlet port and said liquid outlet port filtering liquid,
**characterized in that**
an effective length of a filtration portion of said porous hollow fiber substantially capable of filtration is in the range of from 2,0 to 4,5 cm;
the packing rate of the hollow fiber bundle packed in said housing is in the range from 15 to 40 vol. %.

2. The infusion filter according to claim 1, wherein said packing rate is in the range from 15 to 35 vol %.

3. The infusion filter according to claims 1 or 2, wherein the effective length of said hollow fiber is in the range from 2.5 to 3.5 cm.

4. The infusion filter according to claim 3, wherein the effective length of said hollow fiber is in the range from 2.5 to 3.0 cm.

5. The infusion filter according to anyone of claims 1 to 4, wherein an average inner diameter of the hollow fibers forming the hollow fiber bundle is in the range from 100 to 500 $\mu$m and an average thickness of the hollow fibers is in the range from 20 to 200 $\mu$m.

6. The infusion filter according to claim 5, wherein the average inner diameter of the hollow fibers forming the hollow fiber bundle is in the range from 200 to 400 $\mu$m and the average thickness of the hollow fibers is in the range from 50 to 150 $\mu$m.

7. The infusion filter according to anyone of claims 1 to 6, wherein said hollow fiber comprises at least one material selected from the group consisting of polysulfone, polyethersulfone, polypropylene, polyethylene, cellulose, cellulose derivative, polyacrylonitrile, ethylene-vinyl acetate copolymer and ethylene vinyl alcohol.

8. The infusion filter according to anyone of claims 1 to 7, wherein the number of the hollow fibers packed in said housing is in the range from 10 to 50.

9. The infusion filter according to claim 8, wherein the number of the hollow fibers packed in said housing is in the range from 10 to 30.

10. The infusion filter according to anyone of claims 1 to 9, wherein the amount of liquid filled in said housing is 3.0 ml or less.

11. The infusion filter according to claim 10, wherein the amount of liquid filled in said housing is in the range from 1.0 to 2.0 ml.

12. The infusion filter according to anyone of claims 1 to 11, wherein said housing is of cylindrical shape having a length of 2.0 to 5.0 cm and an inner diameter of 0.3 to 2.0 cm.

13. The infusion filter according to anyone of claims 1 to 12, wherein to flow rate of filtration through said filtration portion is in the range from 15 to 50 ml/min.

14. The infusion filter according to anyone of claims 1 to 13, wherein the total effective filtration area of said filtration portion is in the range from 10 to 40 cm$^2$.

15. The infusion filter according to anyone of claims 1 to 14, wherein the hollow fibers packed in said housing have substantially the same length.

**Patentansprüche**

1. Infusionsfilter umfassend ein Gehäuse mit einer Einführungsöffnung und einer Auslassöffnung für Flüssigkeit, wobei das Gehäuse mit einem Bündel poröser Hohlfasern gepackt ist, wovon das Äußere der beiden Enden mit Keramikmaterialien befestigt ist und wobei die porösen Hohlfasern zwischen der Einführungsöffnung und der Auslassöffnung für Flüssigkeit zum Filtern der Flüssigkeit angeordnet sind, **dadurch gekennzeichnet, dass** eine wirksame Länge eines Filtrierabschnittes der porösen Hohlfaser der im Wesentlichen zum Filtrieren fähig ist, im Bereich von 2,0 bis 4,5 cm liegt und die Packungsrate des Hohlfaserbündels, das in dem Gehäuse gepackt ist, im Bereich von 15 bis 40 Vol% liegt.

**2.** Infusionsfilter nach Anspruch 1, worin die Packungsrate im Bereich von 15 bis 35 Vol% liegt.

**3.** Infusionsfilter nach Anspruch 1 oder 2, worin die wirksame Länge der Hohlfaser im Bereich von 2,5 bis 3,5 cm liegt.

**4.** Infusionsfilter nach Anspruch 3, worin die wirksame Länge der Hohlfaser im Bereich von 2,5 bis 3,0 cm liegt.

**5.** Infusionsfilter nach jedem der Ansprüche 1 bis 4, worin ein mittlerer Innendurchmesser der Hohlfasern, welche das Hohlfaserbündel bilden, im Bereich von 100 bis 500 μm liegt und eine mittlere Dicke der Hohlfasern im Bereich von 20 bis 200 μm liegt.

**6.** Infusionsfilter nach Anspruch 5, worin der mittlere Innendurchmesser der Hohlfasern, welche das Hohlfaserbündel bilden, im Bereich von 200 bis 400 μm liegt und die mittlere Dicke der Hohlfasern im Bereich von 50 bis 150 μm liegt.

**7.** Infusionsfilter nach jedem der Ansprüche 1 bis 6, worin die Hohlfaser wenigstens ein Material umfasst, das aus der Gruppe ausgewählt ist, die aus Polysulfon, Polyethersulfon, Polypropylen, Polyethylen, Cellulose, Cellulosederivat, Polyacrylnitril, Ethylenvinylacetat-Copolymer und Ethylenvinylalkohol besteht.

**8.** Infusionsfilter nach jedem der Ansprüche 1 bis 7, worin die Anzahl der Hohlfasern, welche in dem Gehäuse gepackt sind, im Bereich von 10 bis 50 liegt.

**9.** Infusionsfilter nach Anspruch 8, worin die Anzahl der Hohlfasern, welche in dem Gehäuse gepackt sind, im Bereich von 10 bis 30 liegen.

**10.** Infusionsfilter nach jedem der Ansprüche 1 bis 9, worin die Menge an Flüssigkeit, die in das Gehäuse eingefüllt ist, 3,0 ml oder weniger beträgt.

**11.** Infusionsfilter nach Anspruch 10, worin die Menge an Flüssigkeit, die in das Gehäuse eingefüllt ist, im Bereich von 1,0 bis 2,0 ml liegt.

**12.** Infusionsfilter nach jedem der Ansprüche 1 bis 11, worin das Gehäuse zylindrische Form aufweist und eine Länge von 2,0 bis 5,0 cm und einen Innendurchmesser von 0,3 bis 2,0 cm aufweist.

**13.** Infusionsfilter nach jedem der Ansprüche 1 bis 12, worin die Filter-Fließrate durch den Filterabschnitt im Bereich von 15 bis 50 ml/min liegt.

**14.** Infusionsfilter nach jedem der Ansprüche 1 bis 13, worin der gesamte wirksame Filtrationsbereich des Filterabschnittes im Bereich von 10 bis 40 cm$^2$ liegt.

**15.** Infusionsfilter nach jedem der Ansprüche 1 bis 14, worin die Hohlfasern, die in dem Gehäuse gepackt sind, im Wesentlichen die gleiche Länge aufweisen.

**Revendications**

**1.** Un filtre pour infusion comprenant un boîtier comportant une ouverture d'introduction d'un liquide et un ouverture de sortie d'un liquide, ledit boîtier étant rempli par un faisceau de fibres poreuses et creuses, l'extérieur de chacune des extrémités étant fixé au moyen de matières de remplissage, lesdites fibres poreuses et creuses étant disposées entre ladite entrée de liquide et la dite sortie de liquide filtrant le liquide, **caractérisé en ce que** une longueur effective d'une portion de filtration desdites fibres poreuses creuses et substantiellement capable de filtrer est comprise entre 2,0 et 4,5 cm ;
le taux de remplissage du faisceau de fibre creuse introduit situé dans le boîtier est compris entre 15 et 40% en volume.

2.   Le filtre pour infusion selon la revendication 1, **caractérisé en ce que** ledit rapport de remplissage est dans l'intervalle compris entre 15 et 35% en volume.

3.   Le filtre pour infusion selon l'une des revendications 1 ou 2, dans lequel la longueur effective desdites fibres creuses est dans l'intervalle compris entre 2,5 et 3,5 cm.

4.   Le filtre pour infusion selon la revendication 3, dans lequel la longueur effective de ladite fibre creuse est dans l'intervalle compris entre 2,5 et 3,0 cm.

5.   Le filtre pour infusion selon l'une des revendications 1 à 4, dans lequel le diamètre moyen intérieur des fibres creuses formant la faisceau de fibres creuses est dans l'intervalle compris entre 100 et 500 µm et l'épaisseur moyenne des fibres creuses est dans l'intervalle compris entre 20 et 200 µm.

6.   Le filtre pour infusion selon la revendication 5, dans lequel le diamètre moyen intérieur des fibres creuses formant le faisceau de fibres creuses est dans l'intervalle compris entre 200 et 400 µm et l'épaisseur moyenne des fibres creuses est dans l'intervalle compris entre 50 et 150 µm.

7.   Le filtre pour infusion selon l'une quelconque des revendications 1 à 6, dans lequel ladite fibre creuse comprend au moins une matière choisie dans le groupe consistant en du polysulfone, du polyéthersulfone, du polypropylène, du polyéthylène, de la cellulose, un dérivé de la cellulose, du polyacrylonitrile, un copolymère d'éthylène-vinyle acétate et un alcool éthylène vinylique.

8.   Le filtre pour infusion selon l'une quelconque des revendications 1 à 7, dans lequel le nombre de fibres creuses stockées dans ledit boîtier est dans l'intervalle compris entre 10 et 50.

9.   Le filtre pour infusion selon l'une quelconque des revendications 1 à 7, dans lequel le nombre de fibres creuses stockées dans ledit boîtier est dans l'intervalle compris entre 10 et 30.

10.   Le filtre pour infusion selon l'une quelconque des revendications 1 à 9, dans lequel la quantité de liquide introduite dans ledit boîtier est de 3,0 ml ou moins.

11.   Le filtre pour infusion selon la revendication 10, dans lequel la quantité de liquide introduite dans ledit boîtier est dans l'intervalle compris entre 1,0 et 2,0 ml.

12.   Le filtre pour infusion selon l'une quelconque des revendications 1 à 11, dans lequel ledit boîtier est de forme cylindrique ayant une longueur de 2,0 à 5,0 cm et un diamètre intérieur de 0,3 à 2,0 cm.

13.   Le filtre pour infusion selon l'une quelconque des revendications 1 à 12, dans lequel la vitesse de circulation de la filtration à travers ladite partie de filtration est dans l'intervalle compris entre 15 et 50 ml/min.

14.   Le filtre pour infusion selon l'une quelconque des revendications 1 à 13, dans lequel la surface totale effective de filtration de ladite portion de filtration est dans l'intervalle compris entre 10 et 40 cm$^2$.

15.   Le filtre pour infusion selon l'une quelconque des revendications 1 à 14, dans lequel les fibres creuses stockées dans ledit boîtier ont sensiblement la même longueur.

FIG . 1

FIG . 2

FIG . 3

FIG . 4